# EUROPÄISCHE PATENTSCHRIFT

(11) **EP 1 709 049 B1**
(45) Veröffentlichungstag und Bekanntmachung des Hinweises auf die Patenterteilung: **18.08.2010**
(21) Anmeldenummer: 04804004.2
(22) Anmeldetag: 17.12.2004
(51) Int. Cl.: C07D 473/04, C07D 487/04

(54) **BICYCLISCHE IMIDAZOLVERBINDUNGEN, DEREN HERSTELLUNG UND DEREN VERWENDUNG ALS ARZNEIMITTEL**
BICYCLIC IMIDAZOLE COMPOUNDS, THE PRODUCTION THEREOF AND THEIR USE AS MEDICAMENTS
COMPOSES IMIDAZOL BICYCLIQUES, LEUR FABRICATION ET LEUR UTILISATION COMME MEDICAMENTS

(30) Priorität: 23.12.2003 DE 10360835; 24.09.2004 DE 102004046530
(43) Veröffentlichungstag der Anmeldung: 11.10.2006
(73) Patentinhaber: Boehringer Ingelheim International GmbH, 55216 Ingelheim am Rhein (DE); Boehringer Ingelheim Pharma GmbH & Co.KG, 55216 Ingelheim am Rhein (DE)
(72) Erfinder: HIMMELSBACH, Frank, 88441 Mittelbiberach (DE); LANGKOPF, Elke, 88447 Warthausen (DE); ECKHARDT, Matthias, 88400 Biberach (DE); HAUEL, Norbert, 88433 Schemmerhofen (DE); THOMAS, Leo, 88400 Biberach (DE); TADAYYON, Mohammad, SG14 1HQ Hertford (GB)
(74) Vertreter: Hammann, Heinz
(86) Internationale Anmeldenummer: PCT/EP2004/014399
(87) Internationale Veröffentlichungsnummer: WO 2005/063750

(56) Entgegenhaltungen:
- WO-A-02/068420
- WO-A-03/104229
- WO-A-2004/018468
- WO-A-2004/041820
- WO-A-2004/050658
- WO-A-2004/111051

## Beschreibung

Gegenstand der vorliegenden Erfindung sind neue bicyclische Imidazolvefbindungen der allgemeinen Formel 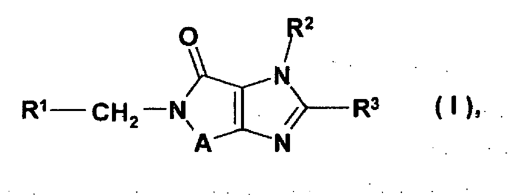 deren Tautomere, Enantiomere, Diastereomere, deren Gemische und deren Salze, insbesonders deren physiologisch verträgliche Salze mit anorganischen oder organischen Säuren, welche wertvolle pharmakologische Eigenschaften aufweisen, insbesondere eine Hemmwirkung auf die Aktivität des Enzyms Dipeptidylpeptidase-IV (DPP-IV), deren Herstellung, deren Verwendung zur Prävention oder Behandlung von Krankheiten oder Zuständen, die in Zusammenhang mit einer erhöhten DPP-IV Aktivität stehen oder die durch Reduktion der DPP-IV Aktivität verhindert oder gemildert werden können, insbesondere von Diabetes mellitus Typ I oder Typ II, die eine Verbindung der allgemeinen Formel (I) oder ein physiologisch verträgliches Salz davon enthaltenden Arzneimittel sowie Verfahren zu deren Herstellung.

Die Dokumente WO 02/068420, WO 03/104229, WO 2004/018468, WO 2004/050658, WO 2004/111051 und WO 2004/041820 beschreiben substituierte bicyclische Heteroaromaten als DPP-IV Hemmer.

### In der obigen Formel I bedeuten

R¹ eine durch die Reste R¹⁰ und R¹¹ substituierte Pyridinyl-, Phenylpyridinyl-, (Pyridinylphenyl)carbonyl-, Chinolinyl-, Phenylchinolinyl-, lsochinolinyl-, Phenylisochinolinyl- oder Phenanthridinyl-Gruppe, wobei das Stickstoffatom der vorstehend erwähnten Gruppen durch ein Sauerstoffatom substituiert ist, und
R¹⁰ und R¹¹, die gleich oder verschieden sein können, ein Wasserstoffatom, ein Fluor-, Chlor- oder Bromatom oder eine Methyl-, Difluormethyl-,Trifluormethyl-, Methoxy-, Difluormethoxy-, Trifluormethoxy- oder Cyano-Gruppe bedeuten,
oder eine durch die Reste R¹⁰ und R¹¹ substituierte Pyrimidinyl-, Phenylpyrimidinyl-, (Pyrimidinylphenyl)carbonyl-, Chinazolinyl-, Phenylchinazolinyl-, Chinoxalinyl-, Phenylchinoxalinyl- oder Naphthyridinyl-Gruppe, wobei mindestens ein Stickstoffatom der vorstehend erwähnten Gruppen durch ein Sauerstoffatom substituiert ist, und R¹⁰ und R¹¹ wie vorstehend erwähnt definiert sind,
R² eine 2-Butin-1-yl-Gruppe,
R³ eine 3-Aminopiperidin-1-yl-, Piperazin-1-yl- oder [1,4]-Diazepan-1-yl-Gruppe, oder eine durch die Reste R⁴ und R⁵ substituierte Aminogruppe, in der
R⁴ eine Methyl- oder Ethyl-Gruppe und
R⁵ eine 2-Aminoethyl-Gruppe bedeuten, wobei der Ethyl-Teil der 2-Aaminoethyl-Gruppe durch eine oder zwei Methylgruppen substituiert sein kann,
und A eine -CO-N(R⁶)- Gruppe, wobei das Stickstoffatom dieser Gruppe mit dem Imidazo-Ring der allgemeinen Formel I verknüpft ist, und
R⁶ eine Methyl-, Ethyl-, Prapyl-, Isopropyl-, Cyclopropyl- oder Phenylgruppe bedeutet, oder eine -N=C(R⁷)- Gruppe, wobei das Kohlenstoffatom dieser Gruppe mit dem Imidazo-Ring der allgemeinen Formel verknüpft ist, und
R⁷ ein Wasserstoffatom oder eine Methylgruppe bedeutet,
deren Tautomere, Enantiomere, Diastereomere, deren Gemische und deren Salze.

Besonders bevorzugt sind diejenigen Verbindungen der allgemeinen Formel I, in denen
R¹ eine durch die Reste R¹⁰ und R¹¹ substituierte Phenylpyridinyl-, Chinolinyl-, Isochinolinyl- oder Phenanthridinyl-Gruppe, wobei das Stickstoffatom der vorstehend erwähnten Gruppen durch ein Sauerstoffatom substituiert ist, und
R¹⁰ ein Wasserstoffatom oder eine Methyl-, Methoxy- oder Cyano-Gruppe und
R¹¹ ein Wasserstoffatom oder eine Methyl-Gruppe bedeuten,
oder eine durch die Reste R¹⁰ und R¹¹ substituierte Phenylpyrimidinyl-, Chinazolinyl-, Chinoxalinyl- oder Naphthyridinyl-Gruppe, wobei mindestens ein Stickstoffatom der vorstehend erwähnten Gruppen durch ein Sauerstoffatom substituiert ist, und R¹⁰ und R¹¹ wie vorstehend erwähnt definiert sind,
R² eine 2-Butin-1-yl-Gruppe,
R³ eine 3-Aminopiperidin-1-yl-, Piperazin-1-yl- oder [1,4]-Diazepan-1-yl-Gruppe, oder
eine durch die Reste R⁴ und R⁵ substituierte Aminogruppe, in der
R⁴ eine Methylgruppe und
R⁵ eine 2-Aminoethyl-Gruppe bedeuten, wobei der Ethyl-Teil der 2-Aminoethyl-Gruppe durch eine oder zwei Methylgruppen substituiert sein kann,
und A eine -CO-N(R⁶)- Gruppe, wobei das Stickstoffatom dieser Gruppe mit dem Imidazo-Ring der allgemeinen Formel I verknüpft ist, und
R⁶ eine Methyl-, Ethyl-, Isopropyl-, Cyclopropyl- oder Phenylgruppe bedeutet,
oder eine -N=C(R⁷)- Gruppe, wobei das Kohlenstoffatom dieser Gruppe mit dem Imidazo-Ring der allgemeinen Formel I verknüpft ist, und
R⁷ ein Wasserstoffatom oder eine Methylgruppe bedeutet,
bedeuten, deren Tautomere, Enantiomere, Diastereomere, deren Gemische und deren Salze.

Eine erste Untergruppe betrifft diejenigen Verbindungen der obigen Formel I, in denen R¹, R² und A wie bereits erwähnt definiert sind und R³ eine 3-Aminopiperidin-1-yl-Gruppe darstellt, deren Tautomere, Enantiomere, Diastereomere, deren Gemische und deren Salze.

Eine zweite Untergruppe betrifft diejenigen Verbindungen der obigen Formel I, in denen R¹, R² und A wie bereits erwähnt definiert sind und R³ eine Piperazin-1-yl-Gruppe darstellt, deren Tautomere, Enantiomere, Diastereomere, deren Gemische und deren Salze.

Eine dritte Untergruppe betrifft diejenigen Verbindungen der obigen Formel I, in denen R¹, R² und A wie bereits erwähnt definiert sind und R³ eine [1,4]-Diazepan-1-yl-Gruppe darstellt, deren Tautomere, Enantiomere, Diastereomere, deren Gemische und deren Salze.

Ganz besonders bevorzugt sind diejenigen Verbindungen der allgemeinen Formel I, in denen
R¹ eine Chinolinyl-, Isochinolinyl-, Methylisochinolinyl- oder Phenanthridinyl-Gruppe, wobei das Stickstoffatom der vorstehend erwähnten Gruppen durch ein Sauerstoffatom substituiert ist,
   eine Chinazolinyl- oder Methylchinazolinyl-Gruppe, wobei ein Stickstoffatom der vorstehend erwähnten Gruppen durch ein Sauerstoffatom substituiert ist,
   oder eine Chinoxalinylgruppe, in der beide Stickstoffatome durch Sauerstoffatome substituiert sind,
R² eine 2-Butin-1-yl-Gruppe,
R³ eine 3-Aminopiperidin-1-yl- oder eine Piperazin-1-yl-Gruppe
und A eine -CO-N(R⁶)- Gruppe, wobei das Stickstoffatom dieser Gruppe mit dem Imidazo-Ring der allgemeinen Formel I verknüpft ist, und R⁶ eine Methylgruppe bedeutet,
oder eine -N=C(R⁷)-Gruppe, wobei das Kohlenstoffatom dieser Gruppe mit dem Imidazo-Ring der allgemeinen Formel I verknüpft ist, und R⁷ ein Wasserstoffatom bedeutet,
bedeuten, deren Tautomere, Enantiomere, Diastereomere, deren Gemische und deren Salze;
insbesondere sind folgende Verbindungen der allgemeinen Formel I zu nennen:
   (a) 1-[(4-Methyl-3-oxy-chinazolin-2-yl)methyl]-3-methyl-7-(2-butin-1-yl)-8-((-R)-3-amino-piperidin-1-yl)-xanthin,
   (b) 1-[(1-Oxy-chinolin-2-yl)methyl]-3-methyl-7-(2-butin-1-yl)-8-((R)-3-amino-piperidin-1-yl)-xanthin,
   (c) 1-[(3-Methyl-2-oxy-isochinolin-1-yl)methyl]-3-methyl-7-(2-butin-1-yl)-8-((*R*)-3-amino-piperidin-1-yl)-xanthin,
   (d) 1-[(5-Oxy-phenanthridin-6-yl)methyl]-3-methyl-7-(2-butin-1-yl)-8-((*R*)-3-amino-piperidin-1-yl)-xanthin,
   (e) 1-[(3-Oxy-chinazolin-2-yl)methyl]-3-methyl-7-(2-butin-1-yl)-8-((*R*)-3-amino-piperidin-1-yl)-xanthin, und
   (f) 2-((*R*)-3-Amino-piperidin-1-yl)-3-(2-butin-1-yl)-5-[(1-oxy-chinolin-2-yl)methyl)]-3,5-dihydro-imidazo[4,5-d]pyridazin-4-on,
sowie deren Tautomere, Enantiomere, Diastereomere, deren Gemische und deren Salze.

Erfindungsgemäß erhält man die Verbindungen der allgemeinen Formel 1 nach an sich bekannten Verfahren, beispielsweise nach folgenden Verfahren:

### a) Umsetzung einer Verbindung der allgemeinen Formel

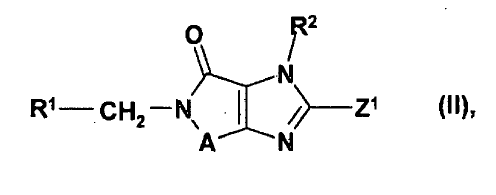

in der
R¹, R² und A wie eingangs erwähnt definiert sind und
Z¹ eine Austrittsgruppe wie ein Halogenatom, eine substituierte Hydroxy-, Mercapto-, Sulfinyl-, Sulfonyl- oder Sulfonyloxygruppe wie ein Chlor- oder Bromatom, eine Methansulfonyl- oder Methensulfonyloxygruppe darstellt, mit R³-H, dessen Enantiomeren oder dessen Salzen, wobei R³ wie eingangs erwähnt definiert ist.

Die Umsetzung wird zweckmäßigerweise in einem Lösungsmittel wie Isopropanol, Butanol, Tetrahydrofuran, Dioxan, Dimethylformamid, Dimethylsulfoxid, Ethylenglycolmonomethylether, Ethylenglycoldiethylether, N-Methyl-pyrrolidin-2-on oder Sulfolan gegebenenfalls in Gegenwart einer anorganischen oder tertiären organischen Base, z.B. Natriumcarbonat, Kaliumcarbonat oder Kaliumhydroxid, einer tertiären organischen Base, z.B. Triethylamin, oder in Gegenwart von N-Ethyl-diisopropylamin (Hünig-Base), wobei diese organischen Basen gleichzeitig auch als Lösungsmittel dienen können, und gegebenenfalls in Gegenwart eines Reaktionsbeschleunigers wie einem Alkalihalogenid oder einem Katalysator auf Palladiumbasis bei Temperaturen zwischen -20 und 180°C, vorzugsweise jedoch bei Temperaturen zwischen -10 und 120°C, durchgeführt. Die Umsetzung kann jedoch auch ohne Lösungsmittel oder in einem Überschuß der Aminoverbindung R³-H durchgeführt werden.

### b) Entschützung einer Verbindung der allgemeinen Formel

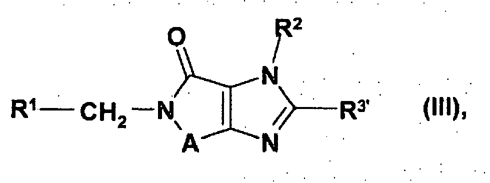

in der R¹, R² und A wie eingangs definiert sind und R^{3'} eine der für R³ eingangs definierten Gruppen darstellt, in denen die Amino- oder Imino-Gruppe durch eine Schutzgruppe wie eine tert.-Butyloxycarbonyl-, Benzyloxycarbonyl-, Formyl- oder Trifluoracetyl-Gruppe geschützt ist, wobei für die Amino-Funktion zusätzlich die Phthalylgruppe in Betracht kommt.

Die Abspaltung des tert.-Butyloxycarbonylrestes erfolgt vorzugsweise durch Behandlung mit einer Säure wie Trifluoressigsäure oder Salzsäure oder durch Behandlung mit Bromtrimethylsilan oder Iodtrimethylsilan gegebenenfalls unter Verwendung eines Lösungsmittels wie Methylenchlorid, Essigester, Dioxan, Methanol, Isopropanol oder Diethylether bei Temperaturen zwischen 0 und 80°C.

Die Abspaltung des Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Temperaturen zwischen 20 und 60°C, und bei einem Wasserstoffdruck von 1 bis 7 bar, vorzugsweise jedoch von 3 bis 5 bar.

Die Abspaltung der Formyl- und der Trifluoracetylgruppe erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid bei Temperaturen zwischen 0 und 120°C, vorzugsweise bei Temperaturen zwischen 10 und 100°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, ToluoI/Wasser oder Dioxan bei Temperaturen zwischen 20 und 50°C.

Bei den vorstehend beschriebenen Umsetzungen können gegebenenfalls vorhandene reaktive Gruppen wie Amino-, Alkylamino- oder Iminogruppen während der Umsetzung durch übliche Schutzgruppen geschützt werden, welche nach der Umsetzung wieder abgespalten werden.

Beispielsweise kommen als Schutzreste für eine Amino-, Alkylamino- oder Imino-gruppe die Formyl-, Acetyl-, Trifluoracetyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Essigsäure/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Natriumhydroxid oder Kaliumhydroxid oder aprotisch, z.B. in Gegenwart von Jodtrimethylsilan, bei Temperaturen zwischen 0 und 120°C, vorzugsweise bei Temperaturen zwischen 10 und 100°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem geeigneten Lösungsmittel wie Methanol, Ethanol, Essigsäureethylester oder Eisessig gegebenenfalls unter Zusatz einer Säure wie Salzsäure bei Temperaturen zwischen 0 und 100°C, vorzugsweise jedoch bei Temperaturen zwischen 20 und 60°C, und bei einem Wasserstoffdruck von 1 bis 7 bar, vorzugsweise jedoch von 3 bis 5 bar. Die Abspaltung eines 2,4-Dimethoxybenzylrestes erfolgt jedoch vorzugsweise in Trifluoressigsäure in Gegenwart von Anisol.

Die Abspaltung eines tert.-Butyl- oder tert.-Butyloxycarbonylrestes erfolgt vorzugsweise durch Behandlung mit einer Säure wie Trifluoressigsäure oder Salzsäure oder durch Behandlung mit Jodtrimethylsilan gegebenenfalls unter Verwendung eines Lösungsmittels wie Methylenchlorid, Dioxan, Methanol oder Diethylether.

Die Abspaltung eines Trifluoracetylrestes erfolgt vorzugsweise durch Behandlung mit einer Säure wie Salzsäure gegebenenfalls in Gegenwart eines Lösungsmittels wie Essigsäure bei Temperaturen zwischen 50 und 120°C oder durch Behandlung mit Natronlauge gegebenenfalls in Gegenwart eines Lösungsmittels wie Tetrahydrofuran bei Temperaturen zwischen 0 und 50°C.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-O-p-toluoyl-weinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure; Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Die als Ausgangsstoffe verwendeten Verbindungen der allgemeinen Formeln II bis III sind entweder literaturbekannt oder man erhält diese nach an sich literaturbekannten Verfahren (siehe Beispiele I bis X).

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf das Enzym DPP-IV.

Die biologischen Eigenschaften der neuen Verbindungen wurden wie folgt geprüft:

Die Fähigkeit der Substanzen und ihrer entsprechenden Salze, die DPP-IV Aktivität zu hemmen, kann in einem Versuchsaufbau gezeigt werden, in dem ein Extrakt der humanen Koloncarcinomzelllinie Caco-2 als DPP IV Quelle benutzt wird. Die Differenzierung der Zellen, um die DPP-IV Expression zu induzieren, wurde nach der Beschreibung von Reiher et al. in einem Artikel mit dem Titel "Increased expression of intestinal cell line Caco-2", erschienen in Proc. Natl. Acad. Sci. Vol. 90, Seiten 5757-5761 (1993), durchgeführt. Der Zellextrakt wurde von in einem Puffer (10 mM Tris HCl, 0.15 M NaCl, 0.04 t.i.u. Aprotinin, 0.5% Nonidet-P40, pH 8.0) solubilisierten Zellen durch Zentrifugation bei 35000 g für 30 Minuten bei 4°C (zur Entfernung von Zelltrümmern) gewonnen.

### Der DPP-IV Assay wurde wie folgt durchgeführt:

50 µl Substratlösung (AFC; AFC ist Amido-4-trifluorinethylcourriarin), Endkonzentration 100 µM, wurden in schwarze Mikrotiterplatten vorgelegt. 20 µl Assay Puffer (Endkonzentrationen 50 mM Tris HCl pH 7.8, 50 mM NaCl, 1 % DMSO) wurde zupipettiert. Die Reaktion wurde durch Zugabe von 30 µl solubilisiertem Caco-2 Protein (Endkonzentration 0.14 µg Protein pro Well) gestartet. Die zu überprüfenden Testsubstanzen wurden typischerweise in 20 µl vorverdünnt zugefügt, wobei das Assaypuffervolumen dann entsprechend reduziert wurde. Die Reaktion wurde bei Raumtemperatur durchgeführt, die Inkubafionsdauer betrug 60 Minuten. Danach wurde die Fluoreszenz in einem Victor 1420 Multilabel Counter gemessen, wobei die Anregungswellenlänge bei 405 nm und die Emissionswellenlänge bei 535 nm lag. Leerwerte (entsprechend 0 % Aktivität) wurden in Ansätzen ohne Caco-2 Protein (Volumen ersetzt durch Assay Puffer), Kontrollwerte (entsprechend 100 % Aktivität) wurden in Ansätzen ohne Substanzzusatz erhalten. Die Wirkstärke der jeweiligen Testsubstanzen, ausgedrückt als IC₅₀ Werte, wurden aus Dosis-Wirkungs Kurven berechnet, die aus jeweils 11 Meßpunkten bestanden. Hierbei wurden folgende Ergebnisse erhalten:

| Verbindung | DPP IV-Hemmung |
|---|---|
| (Beispiel Nr.) | IC₅₀ [nM] |
| 1 | 2 |
| 1(1) | 1 |
| 1(2) | 4 |
| 1(3) | 6 |
| 1(4) | 2 |

Die erfindungsgemäß hergestellten Verbindungen sind gut verträglich, da beispielsweise nach oraler Gabe von 10 mg/kg der Verbindung des Beispiels 1 an Ratten keine Änderungen im Verhalten der Tiere beobachtet werden konnten.

Im Hinblick auf die Fähigkeit, die DPP-IV Aktivität zu hemmen, sind die erfindungsgemäßen Verbindungen der allgemeinen Formel I und ihre entsprechenden pharmazeutisch akzeptablen Salze geeignet, alle diejenigen Zustände oder Krankheiten zu beeinflussen, die durch eine Hemmung der DPP-IV Aktivität beeinflusst werden können. Es ist daher zu erwarten, daß die erfindungsgemäßen Verbindungen zur Prävention oder Behandlung von Krankheiten oder Zuständen wie Diabetes mellitus Typ 1 und Typ 2, Prädiabetes, Verminderung der Glukosetoleranz oder Veränderungen im Nüchternblutzucker, diabetische Komplikationen (wie z.B. Retinopathie, Nephropathie oder Neuropathien), metabolische Azidose oder Ketose, reaktiver Hypoglykämie, Insulinresistenz, Metabolischem Syndrom, Dyslipidämien unterschiedlichster Genese, Arthritis, Atherosklerose und verwandte Erkrankungen, Adipositas, Allograft Transplantation und durch Calcitonin verursachte Osteoporose geeignet sind. Darüberhinaus sind diese Substanzen geeignet, die B-Zelldegeneration wie z.B. Apoptose oder Nekrose von pankreatischen B-Zellen zu verhindern. Die Substanzen sind weiter geeignet, die Funktionalität von pankreatischen Zellen zu verbessern oder wiederherzustellen, daneben die Anzahl und Größe von pankreatischen B-Zellen zu erhöhen. Zusätzlich und begründet durch die Rolle der Glucagon-Like Peptide, wie z.B. GLP-1 und GLP-2 und deren Verknüpfung mit DPP-IV Inhibition, wird erwartet, daß die erfindungsgemäßen Verbindungen geeignet sind, um unter anderem einen sedierenden oder angstlösenden Effekt zu erzielen, darüberhinaus katabole Zustände nach Operationen oder hormonelle Stressantworten günstig zu beeinflussen oder die Mortalität und Morbidität nach Myokardinfarkt reduzieren zu können. Darüberhinaus sind sie geeignet zur Behandlung von allen Zuständen, die im Zusammenhang mit oben genannten Effekten stehen und durch GLP-1 oder GLP-2 vermittelt sind. Die erfindungsgemäßen Verbindungen sind ebenfalls als Diuretika oder Antihypertensiva einsetzbar und zur Prävention und Behandlung des akuten Nierenversagens geeignet. Weiterhin sind die erfindungsgemäßen Verbindungen zur Behandlung entzündlicher Erkrankungen der Atemwege einsetzbar. Ebenso sind sie zur Prävention und Therapie von chronischen entzündlichen Darmerkrankungen wie z.B. Reizdarmsyndrom (IBS), Morbus Crohn oder Colitis ulcerosa ebenso wie bei Pankreatitis geeignet. Des weiteren wird erwartet, daß sie bei jeglicher Art von Verletzung oder Beeinträchtigung im Gastrointestinaltrakt eingesetzt werden können wie auch z.B. bei Kolitiden und Enteriden. Darüberhinaus wird erwartet, daß DPP-IV Inhibitoren und somit auch die erfindungsgemäßen Verbindungen zur Behandlung der Unfruchtbarkeit oder zur Verbesserung der Fruchtbarkeit beim Menschen oder im Säugetierorganismus verwendet werden können, insbesondere dann, wenn die Unfruchtbarkeit im Zusammenhang mit einer Insulinresistenz oder mit dem polyzystischen Ovarialsyndrom steht. Auf der anderen Seite sind diese Substanzen geeignet, die Motilität der Spermien zu beeinflussen und sind damit als Kontrazeptiva zur Verwendung beim Mann einsetzbar. Des weiteren sind die Substanzen geeignet, Mangelzustände von Wachstumshormon, die mit Minderwuchs einhergehen, zu beeinflussen, sowie bei allen Indikationen sinnvoll eingesetzt werden können, bei denen Wachstumshormon verwendet werden kann. Die erfindungsgemäßen Verbindungen sind auf Grund ihrer Hemmwirkung gegen DPP IV auch geeignet zur Behandlung von verschiedenen Autoimmunerkrankungen wie z.B. rheumatoide Arthritis, Multiple Sklerose, Thyreoditiden und Basedow'scher Krankheit etc.. Darüberhinaus können sie eingesetzt werden bei viralen Erkrankungen wie auch z.B. bei HIV Infektionen, zur Stimulation der Blutbildung, bei benigner Prostatahyperplasie, bei Gingivitiden, sowie zur Behandlung von neuronalen Defekten und neurdegenerativen Erkrankungen wie z.B. Morbus Alzheimer. Beschriebene Verbindungen sind ebenso zu verwenden zur Therapie von Tumoren, insbesondere zur Veränderung der Tumorinvasion wie auch Metastatisierung, Beispiele hier sind die Anwendung bei T-Zell Lymphomen, akuter lymphoblastischer Leukämie, zellbasierende Schilddrüsenkarzinome, Basalzellkarzinome oder Brustkarzinome. Weitere Indikationen sind Schlaganfall, Ischämien verschiedenster Genese, Morbus Parkinson und Migräne. Darüberhinaus sind weitere Indikationsgebiete follikuläre und epidermale Hyperkeratosen, erhöhte Keratinozytenproliferation, Psoriasis, Enzephalomyelitiden, Glomerulonephritiden, Lipodystrophien, sowie psychosomatische, depressive und neuropsychiatrische Erkrankungen verschiedenster Genese.

Die erfindungsgemäßen Verbindungen können auch in Kombination mit anderen Wirkstoffen verwendet werden. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. Gl 262570) und -Antagonisten, PPAR-gamma/alpha Modulatoren (z.B. KRP 297), PPAR-gamma/alpha/delta Modulatoren, AMPK-Aktivatoren, ACC1 und ACC2 Inhibitoren, DGAT-Inhibitoren, SMT3-Rezeptor-Agonisten, 11β-HSD-Inhibitoren, FGF19-Agonisten oder -Mimetika, alpha-Glucosidasehemmer (z.B. Acarbose, Voglibose), andere DPPIV Inhibitoren, alpha2-Antagonisten, Insulin und Insulinanaloga, GLP-1 und GLP-1 Analoga (z.B. Exendin-4) oder Amylin. Daneben SGLT2-Inhibitoren wie T-1095 oder KGT-1251 (869682), Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptor Antagonisten und Inhibitoren der Phosphoenolpyruvatcarboxykinase, der Glykogensynthasekinase oder der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, PPAR-alpha agonisten, PPAR-delta agonisten, ACAT Inhibitoren (z.B. Avasimibe) oder Cholesterolresorptionsinhibitoren wie zum Beispiel Ezetimibe, gallensäurebindende Substanzen wie zum Beispiel Colestyramin, Hemmstoffe des ilealen Gallensäuretransportes, HDL-erhöhende Verbindungen wie zum Beispiel Inhibitoren von CETP oder Regulatoren von ABC1 oder LXRalpha Antagonisten, LXRbeta Agonisten oder LXRalpha/beta Regulatoren oder Wirkstoffe zur Behandlung von Obesitas, wie etwa Sibutramin oder Tetrahydrolipstatin, Dexfenfluramin, Axokine, Antagonisten des Cannbinoid1 Rezeptors, MCH-1 Rezeptorantagonisten, MC4 Rezeptor Agonisten, NPY5 oder NPY2 Antagonisten oder β₃-Agonisten wie SB-418790 oder AD-9677 ebenso wie Agonisten des 5HT2c Rezeptors.

Daneben ist eine Kombination mit Medikamenten zur Beeinflussung des Bluthochdrucks wie z.B. All Antagonisten oder ACE Inhibitoren, Diuretika, β-Blocker, Ca-Antagonisten und anderen oder Kombinationen daraus geeignet.

Die zur Erzielung einer entsprechenden Wirkung erforderliche Dosierung beträgt zweckmäßigerweise bei intravenöser Gabe 1 bis 100 mg, vorzugsweise 1 bis 30 mg, und bei oraler Gabe 1 bis 1000 mg, vorzugsweise 1 bis 100 mg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die nachfolgenden Beispiele sollen die Erfindung näher erläutern:

### Herstellung der Ausgangsverbindungen:

### Beispiel I

### 1-[(4-Methyl-3-oxy-chinazolin-2-yl)methyl]-3-methyl-7-(2-butin-1-yl)-8-[(R)-3-(tert.-butyloxycarbonylamino)-piperidin-1-yl]-xanthin

Ein Gemisch aus 300 mg 3-Methyl-7-(2-butin-1-yl)-8-[(*R*)-3-(tert.-butyloxycarbonyl-amino)-piperidin-1-yl]-xanthin, 151 mg 2-Chlormethyl-4-methyl-chinazolin-3-oxid und 220 mg Kaliumcarbonat in 50 ml Acetonitril wird sieben Minuten in der Mikrowelle bei 170 °C erhitzt. Anschließend wird das Acetonitril abdestilliert und der Kolbenrückstand über eine Kieselgelsäule mit Essigester/Methanol (100:0 auf 90:10) als Laufmittel chromatographiert.
Ausbeute: 121 mg (29 % der Theorie)
R_{f}-Wert: 0.60 (Kieselgel, Essigester/Methanol = 9:1)
Massenspektrum (ESI^{*}): m/z = 589 [M+H]⁺

### Beispiel II

### 3-Methyl-7-(2-butin-1-yl)-8-[(R)-3-(tert.-butyloxycarbonylamino)-piperidin-1-yl]-xanthin

Zu 15.00 g 3-Methyl-7-(2-butin-1-yl)-8-brom-xanthin und 16.00 g Kaliumcarbonat in 100 ml Dimethylsulfoxid werden 11.00 g (*R*)-3-tert.-Butyloxycarbonylamino-piperidin gegeben und die dicke hellbeige Suspension wird vier Stunden mit einem mechanischen Rührer bei ca. 114°C gerührt. Dann werden nochmals 900 mg (*R*)-3-tert.-Butyloxycarbonylamino-piperidin, gelöst in 10 ml Dimethylsulfoxid, zum Reaktionsgemisch gegeben und dieses wird weitere zwei Stunden bei 114°C gerührt. Nach Abkühlung auf Raumtemperatur wird das Reaktionsgemisch mit reichlich Wasser verdünnt. Der entstandene Niederschlag wird gründlich verrieben, bis keine Klumpen mehr vorhanden sind, und absaugt. Der helle Feststoff wird erneut mit Wasser aufgeschlämmt, abgesaugt, mit Wasser und Diethylether nachgewaschen und im Umlufttrockenschrank bei 60°C getrocknet.
Ausbeute: 19.73 g (94% der Theorie)
R_{f}-Wert: 0.64 (Kieselgel, Essigester)
Massenspektrum (ESI⁺): m/z = 417 [M+H]⁺

### Analog Beispiel II werden folgende Verbindungen erhalten:

### (1) 2-[(R)-3-(tert.-Butyloxycarbonylamino)-piperidin-1-yl]-3-(2-butin-1-yl)-3,5-dihydro-imidazo[4,5-d]pyridazin-4-on

(Durchführung in N,N-Dimethylformamid bei 80°C)
R_{f}-Weft: 0.55 (Kieselgel, Essigester)
Massenspektrum (ESI^{*}): m/z = 387 [M+H]⁺

### Beispiel III

### 3-Methyl-7-(2-butin-1-yl)-8-brom-xanthin

Zu 30.17 g 3-Methyl-8-brom-xanthin und 27.00 ml Hünigbase in 370 ml N,N-Dimethylformamid werden 17.06 g 1-Brom-2-butin gegeben. Das Reaktionsgemisch wird zwei Stunden bei Raumtemperatur gerührt, dann wird nochmals 1 ml 1-Brom-2-butin nachgesetzt und eine weitere Stunde bei Raumtemperatur gerührt. Zur Aufarbeitung wird das Reaktionsgemisch mit ca. 300 ml Wasser verdünnt. Der entstandene helle Niederschlag wird abgesaugt und mit Wasser nachgewaschen. Der Filterkuchen wird mit wenig Ethanol und Diethylether gewaschen und im Umlufttrockenschrank bei 60°C getrocknet.
Ausbeute: 30.50 g (84 % der Theorie)
R_{f}-Wert: 0.24 (Kieselgel, Methylenchlorid/Methanol = 95:5)
Massenspektrum (ESI⁺): m/z = 297, 299 [M+H]⁺

### Beispiel IV

### 1-[(1-Oxy-chinolin-2-yl)methyl]-3-methyl-7-(2-butin-1-yl)-8-[(R)-3-(tert.-buyloxy-carbonylamino)-piperidin-1-yl]-xanthin

Hergestellt durch Erhitzen von 450 mg 3-Methyl-7-(2-butin-1-yl)-8-[(*R*)-3-(tert.-butyl-oxycarbonylamino)-piperidin-1-yl]-xanthin, 245 mg 2-Chlormethyl-chinolin-1-oxid und 800 mg Kaliumcarbonat in 5 ml N,N-Dimethylformamid auf 80 °C.
Ausbeute: 622 mg (100 % der Theorie)
R_{f}-Wert: 0.26 (Kieselgel, Essigester)

### Massenspektrum (ESI⁺): m/z = 574 [M+H]⁺

Analog Beispiel IV werden folgenden Verbindungen erhalten:
(1) 1-[(3-Methyl-2-oxy-isochinolin-1-yl)methyl]-3-methyl-7-(2-butin-1-yl)-8-[(*R*)-3-(tert.-butyloxycarbonylamino)-piperidin-1-yl]-xanthin
   R_{f}-Wert: 0.17 (Kieselgel, Essigester)
   Massenspektrum (ESI⁺): m/z = 588 [M+H]⁺
(2) 1-[(5-Oxy-phenanthridin-6-yl)methyl]-3-methyl-7-(2-butin-1-yl)-8-[(*R*)-3-(tert.-butyl-oxycarbonylamino)-piperidin-1-yl]-xanthin
   R_{f}-Wert: 0.47 (Kieselgel, Essigester)
   Massenspektrum (ESI⁺): m/z = 624 [M+H]⁺
(3) 1-[(3-Oxy-chinazolin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.29 (Kieselgel, Essigester)
   Massenspektrum (ESI⁺): m/z = 575 [M+H]⁺
(4) 1-[(1,4-Dioxy-chinoxalin-2-yl)methyl]-3-methyl-7-(2-butin-1-yl)-8-[(*R*)-3-(tert.-butyloxycarbonylamino)-piperidin-1-yl]-xanthin
   R_{f}-Wert: 0.53 (Kieselgel, Essigester)
   Massenspektrum (ESI⁺): m/z = 591 [M+H]⁺
(5) 2-[(*R*)-3-(tert.-Butyloxycarbonylamino)-piperidin-1-yl]-3-(2-butin-1-yl)-5-[(1-oxychinolin-2-yl)methyl)]-3,5-dihydro-imidazo[4,5-d]pyridazin-4-on
   Massenspektrum (ESI⁺): m/z = 544 [M+H]⁺
(6) 2-[(*R*)-3-(tert.-Butyloxycarbonylamino)-piperidin-1-yl]-3-(2-butin-1-yl)-5-[(3-methyl-2-oxy-isochinolin-1-yl)methyl]-3,5-dihydro-imidazo[4,5-d]pyridazin-4-on
   Massenspektrum (ESI⁺): m/z = 558 [M+H]⁺
(7) 2-[(*R*)-3-(tert.-Butyloxycarbonylamino)-piperidin-1-yl]-3-(2-butin-1-yl)-5-[(5-oxyphenanthridin-6-yl)methyl]-3,5-dihydro-imidazo[4,5-d]pyridazin-4-on
   Massenspektrum (ESI⁺): m/z = 594 [M+H]⁺
(8) 2-[(*R*)-3-(tert.-Butyloxycarbonylamino)-piperidin-1-yl]-3-(2-butin-1-yl)-5-[(3-oxychinazolin-2-yl)methyl]-3,5-dihydro-imidazo[4,5-d]pyridazin-4-on
   Massenspektrum (ESI⁺): m/z = 545 [M+H]⁺
(9) 2-[(*R*)-3-(tert.-Butyloxycarbonylamino)-piperidin-1-yl]-3-(2-butin-1-yl)-5-[(1,4-dioxychinoxalin-2-yl)methyl]-3,5-dihydro-imidazo[4,5-d]pyridazin-4-on
   Massenspektrum (ESI⁺): m/z = 561 [M+H]⁺
(10) 2-Brom-3-(2-butin-1-yl)-5-[(4-methyl-3-oxy-chinazolin-2-yl)methyl]-3,5-dihydroimidazo[4,5-d]pyridazin-4-on
   R_{f}-Wert: 0.30 (Kieselgel, Essigester)
   Massenspektrum (ESI⁺): m/z = 439, 441 [M+H]⁺
(11) 2-[(*R*)-3-(tert.-Butyloxycarbonylamino)-piperidin-1-yl]-3-(2-butin-1-yl)-5-[(4-methyl-3-oxy-chinazolin-2-yl)methyl]-3,5-dihydro-imidazo[4,5-d]pyridazin-4-on
   R_{f}-Wert: 0.40 (Kieselgel, Essigester/Methanol = 9:1)
   Massenspektrum (ESI⁺): m/z = 559 [M+H]⁺
(12) 1-[(2-Oxy-isochinolin-3-yl)methyl]-3-methyl-7-(2-butin-1-yl)-8-[(*R*)-3-(tert.-butyloxycarbonylamino)-piperidin-1-y1]-xanthin
   R_{f}-Wert: 0.35 (Kieselgel, Methylenchlorid/Methanol = 95:5)
   Massenspektrum (ESI⁺): m/z = 574 [M+H]⁺
(13) 2-[(*R*)-3-(tert.-Butyloxycarbonylamino)-piperidin-1-yl]-3-(2-butin-1-yl)-5-(2-oxy-isochinolin-3-yl)methyl]-3,5-dihydro-imidazo[4,5-d]pyridazin-4-on
   R_{f}-Wert: 0.10 (Kieselgel, Essigester/Methanol = 98:2)

### Massenspektrum (ESI⁺): m/z = 544 [M+H]⁺

### Beispiel V

### 1-Chlomethyl-3-methyl-isochinolin-2-oxid

Eine Lösung aus 300 mg 1-Chlormethyl-3-methyl-isochinolin in 3 ml Methylenchlorid wird mit 390 mg 3-Chlorperoxybenzoesäure versetzt und über Nacht bei Raumtemperatur gerührt. Das Reaktionsgemisch wird mit etwas Methylenchlorid verdünnt und mit gesättigter Natriumhydrogencarbonat-Lösung ausgeschüttelt. Die vereinigten organischen Phasen werden über Magnesiumsulfat getrocknet und eingeengt. Das feste, gelbliche Rohprodukt wird mit tert.-Butylmethylether verrieben, abgesaugt, mit tert.-Butylmethylether nachgewaschen und getrocknet.
Ausbeute: 285 mg (88 % der Theorie)
R_{f}-Wert: 0.31 (Kieselgel, Essigester/Petrolether = 3:2)
Massenspektrum (ESI⁺): m/z = 208, 210 [M+H]⁺

### Analog Beispiel V wird folgende Verbindung erhalten:

### (1) 6-Chlormethyl-phenanthridin-5-oxid

R_{f}-Wert: 0.66 (Kieselgel, Essigester/Petrolether = 3:2)
Massenspektrum (ESI⁺): m/z = 244, 246 [M+H]⁺

### Beispiel VI

### 2-Brommethyl-chinazolin-3-oxid

Eine Lösung von 1.00 g 2-Methyl-chinazolin-3-oxid in 30 ml Eisessig wird tropfenweise mit einer Lösung von 0.48 ml Brom in 10 ml Eisessig versetzt und über Nacht bei Raumtemperatur gerührt. Anschließend wird das Reaktionsgemisch noch zwei Stunden bei 80 °C gerührt. Der Eisessig wird größtenteils abdestilliert und der Rückstand mit gesättigter Natriumhydrogencarbonat-Lösung verrührt. Der ausgefallene, klumpige Niederschlag wird in Essigester aufgenommen. Der Essigester wird wieder abdestilliert und der feine Niederschlag abgesaugt, mit Ethanol und tert.-Butylmethylether gewaschen und getrocknet. Das Rohprodukt wird chromatographisch über eine Kieselgelsäule mit Essigester als Laufmittel gereinigt.
Ausbeute: 654 mg (44 % der Theorie)
R_{f}-Wert: 0.52 (Kieselgel, Essigester)
Massenspektrum (ESI⁺): m/z = 239, 241 [M+H]⁺

### Beispiel VII

### 2-Brom-3-(2-butin-1-yl)-3,5-dihydro-imidazo[4,5-d]pyridazin-4-on

Zu einer Lösung von 1.80 g 2-Brom-3-(2-butin-1-yl)-5-formyl-3H-imidazol-4-carbonsäure-methylester in 25 ml Ethanol werden bei Raumtemperatur 0.31 ml Hydrazinhydrat (99%), gelöst in 1 ml Ethanol, zugetropft. Fünf Minuten später werden 1.5 ml konzentrierte Essigsäure zugefügt und das Gemisch wird 30 Minuten zum Rückfluß erhitzt. Nach dem Abkühlen wird der ausgefallene Feststoff abgesaugt, mit 10 ml Ethanol und 20 ml Diethylether gewaschen und getrocknet.
Ausbeute: 1.25 g (74 % der Theorie)
Massenspektrum (ESI⁺): m/z = 267, 269 [M+H]⁺
1H-NMR-Spektrum (d6-DMSO): δ = 1.80 (s, 3H); 5.28 (s, 2H); 8.38 (s, 1H); 12.99 (s, 1H) ppm

### Beispiel VIII

### 2-Brom-3-(2-butin-1-yl)-5-formyl-3H-imidazol-4-carbonsäure-methylester

Zu einer Lösung von 13.5 g 2-Brom-1-(2-butin-1-yl)-1H-imidazol-4,5-dicarbonsäure-dimethylester in 220 ml Tetrahydrofuran werden unter Argon-Atmosphäre bei -70°C 43 ml einer 1 M Lösung von Diisobutyl-aluminiumhydrid in Tetrahydrofuran innerhalb 20 Minuten zugetropft. Es wird weitere vier Stunden bei -70°C gerührt, dann werden 20 ml einer Mischung aus 1 M Salzsäure und Tetrahydrofuran zugetropft. Nach dem Erwärmen auf Raumtemperatur werden ca. 200 ml Wasser hinzugegeben und dreimal mit je 70 ml Essigester extrahiert. Die vereinigten Extrakte werden getrocknet und eingeengt. Das so erhaltene Rohprodukt wird durch Säulenchromatographie über Kieselgel mit Petrolether/Essigester (80:20 auf 50:50) als Laufmittel gereinigt
Ausbeute: 6.40 g (52% der Theorie)
Massenspektrum (ESI⁺): m/z = 285, 287 [M+H]⁺
1H-NMR-Spektrum (d6-DMSO): δ = 1.80 (s, 3H); 3.93 (s, 3H); 5.11 (s, 2H); 10.12 (s, 1H) ppm

### Beispiel IX

### 2-Brom-1-(2-butin-1-yl)-1H-imidazol-4,5-dicarbonsäure-dimethylester

Eine Lösung von 15.0 g 2-Brom-imidazol-4,5-dicarbonsäure-dimethylester, 5.15 ml 1-Brom-2-butin und 50 ml N,N-Diisopropylethylamin in 280 ml Tetrahydrofuran wird eine Stunde zum Rückfluß erhitzt. Das Gemisch wird eingedampft, der Rückstand mit ca. 100 ml Wasser versetzt und dreimal mit je 70 ml Essigester extrahiert. Die Extrakte werden mit 50 ml Wasser gewaschen, getrocknet und eingeengt. Das so erhaltene Rohprodukt wird durch Säulenchromatographie über Kieselgel mit Methylenchlorid/Ethanol (100:0 auf 98:2) als Laufmittel gereinigt.
Ausbeute: 13.50 g (75 % der Theorie)
R_{f}-Wert: 0.82 (Kieselgel, Methylenchlorid/Ethanol = 9:1)
Massenspektrum (ESI⁺): m/z = 315, 317 [M+H]⁺

### Beispiel X

### 3-Chlormethyl-isochinolin-2-oxid

Hergestellt durch Behandeln von 3-Chlormethyl-isochinolin mit 35 %iger Wasserstoffperoxid-Lösung in Eisessig bei 70°C.
R_{f}-Wert: 0.30 (Kieselgel, Essigester/Methanol = 98:2)
Massenspektrum (ESI⁺): m/z = 194, 196 [M+H]⁺

### Herstellung der Endverbindungen:

### Beispiel 1

### 1-[(4-Methyl-3-oxy-chinazolin-2-yl)methyl]-3-methyl-7-(2-butin-1-yl)-8-((R)-3-amino-piperidin-1-yl)-xanthin

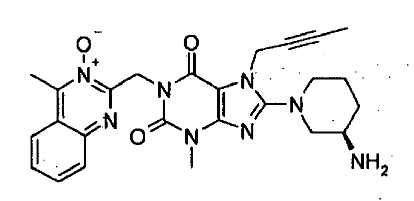

Ein Gemisch aus 121 mg 1-[(4-Methyl-3-oxy-chinazolin-2-yl)methyl]-3-methyl-7-(2-butin-1-yl)-8-[(*R*)-3-(tert.-butyloxycarbonylamino)-piperidin-1-yl]-xanthin und 0.59 ml Trifluoressigsäure in 4 ml Methylenchlorid wird eine Stunde bei Raumtemperatur gerührt. Zur Aufarbeitung wird das Reaktionsgemisch mit Methylenchlorid und Wasser verdünnt, mit 1 N Natronlauge alkalisch gestellt und mit Methylenchlorid extrahiert. Die organische Phase wird mit gesättigter Natriumchlorid-Lösung gewaschen, über Magnesiumsulfat getrocknet und eingeengt. Es bleibt ein bräunlicher Feststoff zurück.
Ausbeute: 84 mg (84 % der Theorie)
R_{f}-Wert: 0.50 (Reversed Phase DC-Fertigplatte (E. Merck), Acetonitril/Wasser/Trifluoressigsäure = 50:50:1)
Massenspektrum (ESI⁺): m/z = 489 [M+H]⁺

### Analog Beispiel 1 werden folgende Verbindungen erhalten:

### (1) 1-[(1-Oxy-chinolin-2-yl)methyl]-3-methyl-7-(2-butin-1-yl)-8-((R)-3-amino-piperidin-1-yl)-xanthin

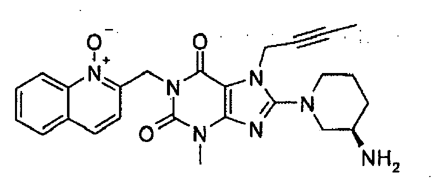

### (Durchführung mit isopropanolischer Salzsäure (5-6 M) in Methylenchlorid)

R_{f}-Wert: 0.53 (Kieselgel, Methylenchlorid/Methanol/konz. wässriges Ammoniak = 90:10:1)
Massenspektrum (ESI⁺): m/z = 474 [M+H]⁺

### (2) 1-[(3-Methyl-2-oxy-isochinolin-1-yl)methyl]-3-methyl-7-(2-butin-1-yl)-8-((R)-3-amino-piperidin-1-yl)-xanthin

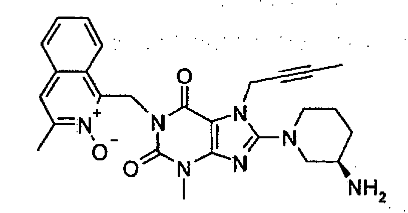

### (Durchführung mit isopropanolischer Salzsäure (5-6 M) in Methylenchlorid)

R_{f}-Wert: 0.39 (Kieselgel, Methylenchlorid/Methanol/konz. wässriges Ammoniak = 90:10:1)
Massenspektrum (ESI⁺): m/z = 488 [M+H]⁺

### (3) 1-[(5-Oxy-phenanthridin-6-yl)methyl]-3-methyl-7-(2-butin-1-yl)-8-((R)-3-amino-piperidin-1-yl)-xanthin

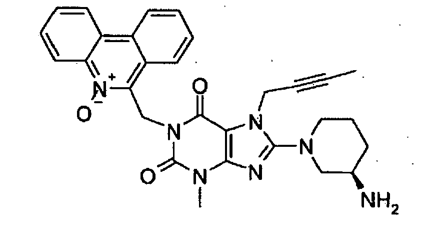

### (Durchführung mit isopropanolischer Salzsäure (5-6 M) in Methylenchlorid)

R_{f}-Wert: 0.47 (Kieselgel, Methylenchlorid/Methanol/konz. wässriges Ammoniak = 90:10:1)
Massenspektrum (ESI⁺): m/z = 524 [M+H]⁺

### (4) 1-[(3-Oxy-chinazolin-2-yl)methyl]-3-methyl-7-(2-butin-1-yl)-8-((R)-3-amino-piperidin-1-yl)-xanthin

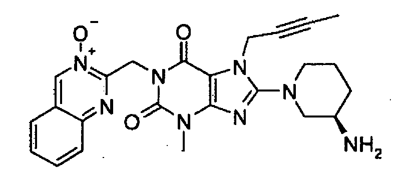

### (Durchführung mit isopropanolischer Salzsäure (5-6 M) in Methylenchlorid)

R_{f}-Wert: 0.41 (Kieselgel, Methylenchlorid/Methanol/konz. wässriges Ammoniak = 90:10:1)
Massenspektrum (ESI⁺): m/z = 475 [M+H]⁺

### (5) 1-[(1,4-Dioxy-chinoxalin-2-yl)methyl]-3-methyl-7-(2-butin-1-yl)-8-((R)-3-amino-piperidin-1-yl)-xanthin

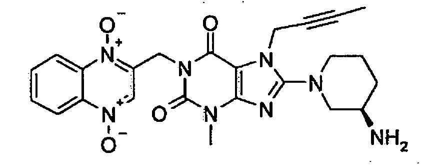

### (Durchführung mit isapropanolischer Salzsäure (5-6 M) in Methylenchlorid)

R_{f}-Wert: 0.55 (Kieselgel, Methylenchlorid/Methanol/konz. wässriges Ammoniak = 90:10:1)
Massenspektrum (ESI⁺): mlz = 491 [M+H]⁺

### (6) 2-((R)-3-Amino-piperidin-1-yl)-3-(2-butin-1-yl)-5-[(1-oxy-chinolin-2-yl)methyl]-3,5-dihydro-imidazo[4,5-d]pyridazin-4-on

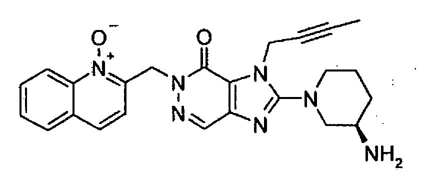

### (Durchführung mit isopropanolischer Salzsäure (5-6 M) in Methylenchlorid)

R_{f}-Wert: 0.33 (Kieselgel, Methylenchlorid/Methanol/konz. wässriges Ammoniak = 90:10:1)
Massenspektrum (ESI⁺): m/z = 444 [M+H]⁺

### (7) 2-((R)-3-Amino-piperidin-1-yl)-3-(2-butin-1-yl)-5-[(3-methyl-2-oxy-isochinolin-1-yl)methyl]-3,5-dihydro-imidazo[4,5-d]pyridazin-4-on

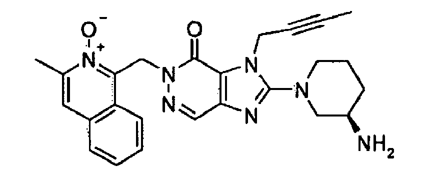

R_{f}-Wert: 0.27 (Kieselgel, Methylenchlorid/Methanol/konz. wässriges Ammoniak = 90:10:1)
Massenspektrum (ESI⁺): m/z = 458 [M+H]⁺

### (8) 2-((R)-3-Amino-piperidin-1-yl)-3-(2-butin-1-yl)-5-[(5-oxy-phenanthridin-6-yl)methyl]-3,5-dihydro-imidazo[4,5-d]pyridazin-4-on

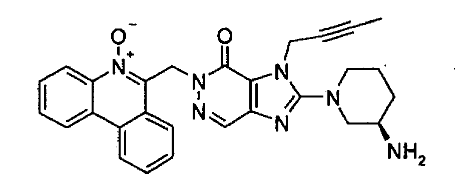

### (Durchführung mit isopropanolischer Salzsäure (5-6 M) in Methylenchlorid)

R_{f}-Wert: 0.24 (Kieselgel, Methylenchlorid/Methanol/konz. wässriges Ammoniak = 90:10:1)
Massenspektrum (ESI⁺: m/z = 494 [M+H]⁺

### (10) 2-((R)-3-Amino-piperidin-1-yl)-3-(2-butin-1-yl)-5-[(1,4-dioxy-chinoxalin-2-yl)methyl]-3,5-dihydro-imidazo[4,5-d]pyridazin-4-on

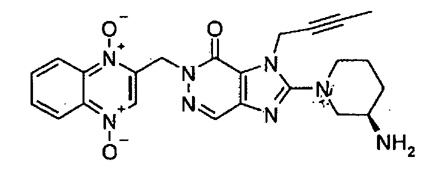

R_{f}-Wert: 0.26 (Kieselgel, Methylenchlorid/Methanol/konz. wässriges Ammoniak = 90:10:1)
Massenspektrum (ESI⁺): m/z = 461 [M+H]⁺

### Beispiel 3

### Dragées mit 75 mg Wirksubstanz

| 1 Dragéekern enthält: | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Dragéekerne werden mit einem Film überzogen, der im wesentlichen aus Hydroxypropylmethylcellulose besteht. Die fertigen Filmdragées werden mit Bienenwachs geglänzt.
Dragéegewicht: 245 mg.

### Beispiel 4

### Tabletten mit 100 mg Wirksubstanz

Zusammensetzung:

| 1 Tablette enthält: | |
|---|---|
| Wirksubstanz | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.

| | |
|---|---|
| Tablettengewicht: | 220 mg |
| Durchmesser: | 10 mm, biplan mit beidseitiger Facette und einseitiger Teilkerbe. |

### Beispiel 5

### Tabletten mit 150 mg Wirksubstanz

Zusammensetzung:

| 1 Tablette enthält: | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1,0 mg |
| | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden
Tabletten gepreßt.

| | |
|---|---|
| Tablettengewicht: | 300 mg |
| Stempel: | 10 mm, flach |

### Beispiel 6

### Hartgelatine-Kapseln mit 150 mg Wirksubstanz

| 1 Kapsel enthält: | |
|---|---|
| Wirkstoff | 150,0 mg |
| Maisstärke getr. | ca. 180,0 mg |
| Milchzucker pulv. | ca. 87,0 mg |
| Magnesiumstearat | 3,0 mg |
| | ca. 420,0 mg |

### Herstellung:

Der Wirkstoff wird mit den Hilfsstoffen vermengt, durch ein Sieb von 0,75 mm-Maschenweite gegeben und in einem geeigneten Gerät homogen gemischt. Die Endmischung wird in Hartgelatine-Kapseln der Größe 1 abgefüllt.
Kapselfüllung: ca. 320 mg
Kapselhülle: Hartgelatine-Kapsel Größe 1.

### Beispiel 7

### Suppositorien mit 150 mg Wirksubstanz

### 1 Zäpfchen enthält:

| | |
|---|---|
| Wirkstoff | 150,0 mg |
| Polyethylenglykol 1500 | 550,0 mg |
| Polyethylenglykol 6000 | 460,0 mg |
| Polyoxyethylensorbitanmonostearat | 840,0 mg |
| | 2000,0 mg |

### Herstellung:

Nach dem Aufschmelzen der Suppositorienmasse wird der Wirkstoff darin homogen verteilt und die Schmelze in vorgekühlte Formen gegossen.

### Beispiel 8

### Suspension mit 50 mg Wirksubstanz

100 ml Suspension enthalten:

| | |
|---|---|
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 9

### Ampullen mit 10 mg Wirksubstanz

Zusammensetzung:

| | |
|---|---|
| Wirkstoff | 10,0 mg |
| 0,01 n Salzsäure s.q. | |
| Aqua bidest | ad 2,0 ml |

### Herstellung:

Die Wirksubstanz wird in der erforderlichen Menge 0,01 n HCl gelöst, mit Kochsalz isotonisch gestellt, sterilfiltriert und in 2 ml Ampullen abgefüllt.

### Beispiel 10

### Ampullen mit 50 mg Wirksubstanz

Zusammensetzung:

| | |
|---|---|
| Wirkstoff | 50,0 mg |
| 0,01 n Salzsäure s.q. | |
| Aqua bidest | ad 10,0 ml |

### Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

## Patentansprüche

1. Verbindungen der allgemeinen Formel 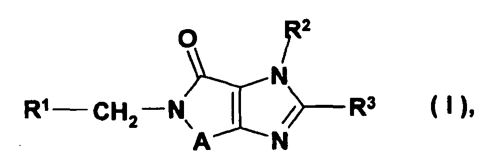 in denen
R¹ eine durch die Reste R¹⁰ und R¹¹ substituierte Pyridinyl-, Phenylpyridinyl-, (Pyridinylphenyl)carbonyl-, Chinolinyl-, Phenylchinolinyl-, Isochinolinyl-, Phenylisochinolinyl- oder Phenanthridinyl-Gruppe, wobei das Stickstoffatom der vorstehend erwähnten Gruppen durch ein Sauerstoffatom substituiert ist, und
R¹⁰ und R¹¹, die gleich oder verschieden sein können, ein Wasserstoffatom, ein Fluor-, Chlor- oder Bromatom oder eine Methyl-, Difluormethyl-,Trifluormethyl-, Methoxy-, Difluormethoxy-, Trifluormethoxy- oder Cyano-Gruppe bedeuten,
oder eine durch die Reste R¹⁰ und R¹¹ substituierte Pyrimidinyl-, Phenylpyrimidinyl-, (Pyrimidinylphenyl)carbonyl-, Chinazolinyl-, Phenylchinazolinyl-, Chinoxalinyl-, Phenylchinoxalinyl- oder Naphthyridinyl-Gruppe, wobei mindestens ein Stickstoffatom der vorstehend erwähnten Gruppen durch ein Sauerstoffatom substituiert ist, und R¹⁰ und R¹¹ wie vorstehend erwähnt definiert sind,
R² eine 2-Butin-1-yl-Gruppe,
R³ eine 3-Aminopiperidin-1-yl-, Piperazin-1-yl- oder [1,4]-Diazepan-1-yl-Gruppe, oder
eine durch die Reste R⁴ und R⁵ substituierte Aminogruppe, in der
R⁴ eine Methyl- oder Ethyl-Gruppe und
R⁵ eine 2-Aminoethyl-Gruppe bedeuten, wobei der Ethyl-Teil der 2-Aminoethyl-Gruppe durch eine oder zwei Methylgruppen substituiert sein kann,
und A eine -CO-N(R⁶)- Gruppe, wobei das Stickstoffatom dieser Gruppe mit dem Imidazo-Ring der allgemeinen Formel I verknüpft ist, und
R⁶ eine Methyl-, Ethyl-, Propyl-, Isopropyl-, Cyclopropyl- oder Phenylgruppe bedeutet,
oder eine -N=C(R⁷)- Gruppe, wobei das Kohlenstoffatom dieser Gruppe mit dem Imidazo-Ring der allgemeinen Formel I verknüpft ist, und
R⁷ ein Wasserstoffatom oder eine Methylgruppe bedeutet,
bedeuten, deren Tautomere, Enantiomere, Diastereomere, deren Gemische und deren Salze.

2. Verbindungen der allgemeinen Formel I gemäß Anspruch 1, in denen
R¹ eine durch die Reste R¹⁰ und R¹¹ substituierte Phenylpyridinyl-, Chinolinyl-, Isochinolinyl- oder Phenanthridinyl-Gruppe, wobei das Stickstoffatom der vorstehend erwähnten Gruppen durch ein Sauerstoffatom substituiert ist, und
R¹⁰ ein Wasserstoffatom oder eine Methyl-, Methoxy- oder Cyano-Gruppe und
R¹¹ ein Wasserstoffatom oder eine Methyl-Gruppe bedeuten,
oder eine durch die Reste R¹⁰ und R¹¹ substituierte Phenylpyrimidinyl-, Chinazolinyl-, Chinoxalinyl- oder Naphthyridinyl-Gruppe, wobei mindestens ein Stickstoffatom der vorstehend erwähnten Gruppen durch ein Sauerstoffatom substituiert ist, und R¹⁰ und R¹¹ wie vorstehend erwähnt definiert sind,
R² eine 2-Butin-1-yl-Gruppe,
R³ eine 3-Aminopiperidin-1-yl-, Piperazin-1-yl- oder [1,4]-Diazepan-1-yl-Gruppe, oder
eine durch die Reste R⁴ und R⁵ substituierte Aminogruppe, in der
R⁴ eine Methylgruppe und
R⁵ eine 2-Aminoethyl-Gruppe bedeuten, wobei der Ethyl-Teil der 2-Aminoethyl-Gruppe durch eine oder zwei Methylgruppen substituiert sein kann,
und A eine -CO-N(R⁶)- Gruppe, wobei das Stickstoffatom dieser Gruppe mit dem Imidazo-Ring der allgemeinen Formel I verknüpft ist, und
R⁶ eine Methyl-, Ethyl-, Isopropyl-, Cyclopropyl- oder Phenylgruppe bedeutet,
oder eine -N=C(R⁷)- Gruppe, wobei das Kohlenstoffatom dieser Gruppe mit dem Imidazo-Ring der allgemeinen Formel I verknüpft ist, und
R⁷ ein Wasserstoffatom oder eine Methylgruppe bedeutet,
bedeuten, deren Tautomere, Enantiomere, Diastereomere, deren Gemische und deren Salze.

3. Verbindungen nach mindestens einem der Ansprüche 1 bis 2, in denen R¹, R² und A wie in den Ansprüchen 1 bis 2 erwähnt definiert sind und R³ eine 3-Aminopiperidin-1-yl-Gruppe darstellt, deren Tautomere, Enantiomere, Diastereomere, deren Gemische und deren Salze.

4. Verbindungen nach mindestens einem der Ansprüche 1 bis 2, in denen R¹, R² und A wie in den Ansprüchen 1 bis 2 erwähnt definiert sind und R³ eine Piperazin-1-yl-Gruppe darstellt, deren Tautomere, Enantiomere, Diastereomere, deren Gemische und deren Salze.

5. Verbindungen nach mindestens einem der Ansprüche 1 bis 2, in denen R¹, R² und A wie in den Ansprüchen 1 bis 2 erwähnt definiert sind und R³ eine [1,4]-Diazepan-1-yl-Gruppe darstellt, deren Tautomere, Enantiomere, Diastereomere, deren Gemische und deren Salze.

6. Verbindungen der allgemeinen Formel I gemäß Anspruch 1, in denen
R¹ eine Chinolinyl-, Isochinolinyl-, Methylisochinolinyl- oder Phenanthridinyl-Gruppe, wobei das Stickstoffatom der vorstehend erwähnten Gruppen durch ein Sauerstoffatom substituiert ist,
eine Chinazolinyl- oder Methylchinazolinyl-Gruppe, wobei ein Stickstoffatom der vorstehend erwähnten Gruppen durch ein Sauerstoffatom substituiert ist,
oder eine Chinoxalinylgruppe, in der beide Stickstoffatome durch Sauerstoffatome substituiert sind,
R² eine 2-Butin-1-yl-Gruppe,
R³ eine 3-Aminopiperidin-1-yl- oder eine Piperazin-1-yl-Gruppe
und A eine -CO-N(R⁶)- Gruppe, wobei das Stickstoffatom dieser Gruppe mit dem Imidazo-Ring der allgemeinen Formel I verknüpft ist, und R⁶ eine Methylgruppe bedeutet,
oder eine -N=C(R⁷)-Gruppe, wobei das Kohlenstoffatom dieser Gruppe mit dem Imidazo-Ring der allgemeinen Formel I verknüpft ist, und R⁷ ein Wasserstoffatom bedeutet,
bedeuten, deren Tautomere, Enantiomere, Diastereomere, deren Gemische und deren Salze.

7. Folgende Verbindungen der allgemeinen Formel I gemäß Anspruch 1:
(a) 1-[(4-Methyl-3-oxy-chinazolin-2-yl)methyl]-3-methyl-7-(2-butin-1-yl)-8-((R)-3-amino-piperidin-1-yl)-xanthin,
(b) 1-[(1-Oxy-chinolin-2-yl)methyl]-3-methyl-7-(2-butin-1-yl)-8-((R)-3-amino-piperidin-1-yl)-xanthin,
(c) 1-[(3-Methyl-2-oxy-isochinolin-1-yl)methyl]-3-methyl-7-(2-butin-1-yl)-8-((*R*)-3-amino-piperidin-1-yl)-xanthin,
(d) 1-[(5-Oxy-phenanthridin-6-yl)methyl]-3-methyl-7-(2-butin-1-yl)-8-((R)-3-amino-piperidin-1-yl)-xanthin,
(e) 1-[(3-Oxy-chinazolin-2-yl)methyl]-3-methyl-7-(2-butin-1-yl)-8-((*R*)-3-amino-piperidin-1-yl)-xanthin,
(f) 2-((*R*)-3-Amino-piperidin-1-yl)-3-(2-butin-1-yl)-5-[(1-oxy-chinolin-2-yl)methyl)]-3,5-dihydro-imidazo[4,5-d]pyridazin-4-on,
sowie deren Tautomere, Enantiomere, Diastereomere, deren Gemische und deren Salze.

8. Physiologisch verträgliche Salze der Verbindungen nach mindestens einem der Ansprüche 1 bis 7 mit anorganischen oder organischen Säuren.

9. Arzneimittel, enthaltend eine Verbindung nach mindestens einem der Ansprüche 1 bis 7 oder ein physiologisch verträgliches Salz gemäß Anspruch 8 neben gegebenenfalls einem oder mehreren inerten Trägerstoffen und/oder Verdünnungsmitteln.

10. Verwendung einer Verbindung nach mindestens einem der Ansprüche 1 bis 8 zur Herstellung eines Arzneimittels, das zur Behandlung von Diabetes mellitus Typ I und Typ II, Arthritis, Adipositas, Allograft Transplantation und durch Calcitonin verursachte Osteoporose geeignet ist.

11. Verfahren zur Herstellung eines Arzneimittels gemäß Anspruch 9, **dadurch gekennzeichnet, daß** auf nichtchemischen Weg eine Verbindung nach mindestens einem der Ansprüche 1 bis 8 in einen oder mehrere inerte Trägerstoffe und/oder Verdünnungsmittel eingearbeitet wird.

12. Verfahren zur Herstellung der Verbindungen der allgemeinen Formel I gemäß den Ansprüchen 1 bis 8, **dadurch gekennzeichnet, daß**
a) eine Verbindung der allgemeinen Formel 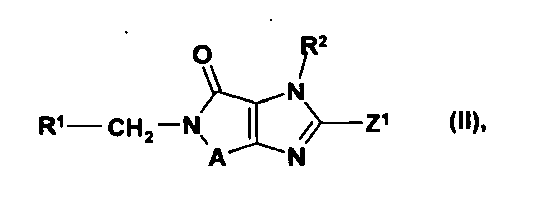 in der
R¹, R² und A wie in den Ansprüchen 1 bis 7 erwähnt definiert sind und
Z¹ eine Austrittsgruppe wie ein Halogenatom, eine substituierte Hydroxy-, Mercapto-, Sulfinyl-, Sulfonyl- oder Sulfonyloxygruppe darstellt,
mit R³-H, dessen Enantiomeren oder dessen Salzen umgesetzt wird, wobei R³ wie eingangs erwähnt definiert ist, oder
b) eine Verbindung der allgemeinen Formel 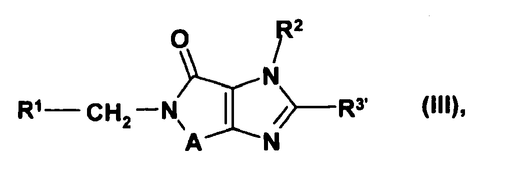 in der R¹, R² und A wie in den Ansprüchen 1 bis 7 erwähnt definiert sind, und R³ eine der für R³ eingangs definierten Gruppen darstellt, in denen die Amino- oder Imino-Gruppe durch eine Schutzgruppe geschützt ist, entschützt wird,
und/oder
anschließend gegegebenenfalls während der Umsetzung verwendete Schutzgruppen abgespalten werden und/oder
die so erhaltenen Verbindungen der allgemeinen Formel I in ihre Enantiomeren und/oder Diastereomeren aufgetrennt werden und/oder
die erhaltenen Verbindungen der Formel I in ihre Salze, insbesondere für die pharmazeutische Anwendung in ihre physiologisch verträglichen Salze mit anorganischen oder organischen Säuren, übergeführt werden.

## Claims

1. Compounds of general formula 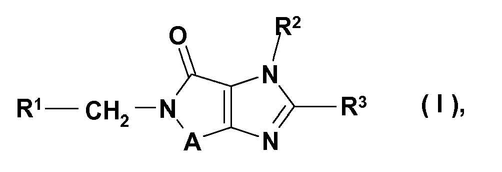 wherein
R¹ denotes a pyridinyl, phenylpyridinyl, (pyridinylphenyl)carbonyl, quinolinyl, phenylquinolinyl, isoquinolinyl, phenylisoquinolinyl or phenanthridinyl group substituted by the groups R¹⁰ and R¹¹, wherein the nitrogen atom of the above-mentioned groups is substituted by an oxygen atom, and
R¹⁰ and R¹¹, which may be identical or different, denote a hydrogen atom, a fluorine, chlorine or bromine atom or a methyl, difluoromethyl, trifluoromethyl, methoxy, difluoromethoxy, trifluoromethoxy or cyano group,
or a pyrimidinyl, phenylpyrimidinyl, (pyrimidinylphenyl)carbonyl, quinazolinyl, phenylquinazolinyl, quinoxalinyl, phenylquinoxalinyl or naphthyridinyl group substituted by the groups R¹⁰ and R¹¹, wherein at least one nitrogen atom of the above-mentioned groups is substituted by an oxygen atom, and R¹⁰ and R¹¹ are as hereinbefore defined,
R² denotes a 2-butyn-1-yl group,
R³ denotes a 3-aminopiperidin-1-yl, piperazin-1-yl or [1,4]-diazepan-1-yl group, or
an amino group substituted by the groups R⁴ and R⁵ wherein
R⁴ denotes a methyl or ethyl group and
R⁵ denotes a 2-aminoethyl group, wherein the ethyl moiety of the 2-aminoethyl group may be substituted by one or two methyl groups,
and A denotes a -CO-N(R⁶) group, wherein the nitrogen atom of this group is linked to the imidazo ring of general formula I, and
R⁶ denotes a methyl, ethyl, propyl, isopropyl, cyclopropyl or phenyl group,
or an -N=C(R⁷)- group, wherein the carbon atom of this group is linked to the imidazo ring of general formula I, and
R⁷ denotes a hydrogen atom or a methyl group,
the tautomers, the enantiomers, the diastereomers, the mixtures thereof and the salts thereof.

2. Compounds of general formula I according to claim 1, wherein
R¹ denotes a phenylpyridinyl, quinolinyl, isoquinolinyl or phenanthridinyl group substituted by the groups R¹⁰ and R¹¹, wherein the nitrogen atom of the above-mentioned groups is substituted by an oxygen atom, and
R¹⁰ denotes a hydrogen atom or a methyl, methoxy or cyano group and
R¹¹ denotes a hydrogen atom or a methyl group,
or a phenylpyrimidinyl, quinazolinyl, quinoxalinyl or naphthyridinyl group substituted by the groups R¹⁰ and R¹¹, wherein at least one nitrogen atom of the above-mentioned groups is substituted by an oxygen atom, and R¹⁰ and R¹¹ are as hereinbefore defined,
R² denotes a 2-butyn-1-yl group,
R³ denotes a 3-aminopiperidin-1-yl, piperazin-1-yl or [1,4]-diazepan-1-yl group, or
an amino group substituted by the groups R⁴ and R⁵ wherein
R⁴ denotes a methyl group and
R⁵ denotes a 2-aminoethyl group, wherein the ethyl moiety of the 2-aminoethyl group may be substituted by one or two methyl groups,
and A denotes a -CO-N(R⁶)- group, wherein the nitrogen atom of this group is linked to the imidazo ring of general formula I, and
R⁶ denotes a methyl, ethyl, isopropyl, cyclopropyl or phenyl group,
or an -N=C(R⁷)- group, wherein the carbon atom of this group is linked to the imidazo ring of general formula I, and
R⁷ denotes a hydrogen atom or a methyl group ,
the tautomers, the enantiomers, the diastereomers, the mixtures thereof and the salts thereof.

3. Compounds according to at least one of claims 1 to 2, wherein R¹, R² and A are defined as in claims 1 to 2 and R³ denotes a 3-aminopiperidin-1-yl group, the tautomers, the enantiomers, the diastereomers, the mixtures thereof and the salts thereof.

4. Compounds according to at least one of claims 1 to 2, wherein R¹, R² and A are defined as in claims 1 to 2 and R³ denotes a piperazin-1-yl group, the tautomers, the enantiomers, the diastereomers, the mixtures thereof and the salts thereof.

5. Compounds according to at least one of claims 1 to 2, wherein R¹, R² and A are defined as in claims 1 to 2 and R³ denotes a [1,4]-diazepan-1-yl group, the tautomers, the enantiomers, the diastereomers, the mixtures thereof and the salts thereof.

6. Compounds of general formula I according to claim 1, wherein
R¹ denotes a quinolinyl, isoquinolinyl, methylisoquinolinyl or phenanthridinyl group, wherein the nitrogen atom of the above-mentioned groups is substituted by an oxygen atom,
a quinazolinyl or methylquinazolinyl group, wherein the nitrogen atom of the above-mentioned groups is substituted by an oxygen atom,
or a quinoxalinyl group wherein both nitrogen atoms are substituted by oxygen atoms,
R² denotes a 2-butyn-1-yl group,
R³ denotes a 3-aminopiperidin-1-yl or a piperazin-1-yl group
and A denotes a -CO-N(R⁶)- group, wherein the nitrogen atom of this group is linked to the imidazo ring of general formula I, and R⁶ denotes a methyl group,
or an -N=C(R⁷)-group, wherein the carbon atom of this group is linked to the imidazo ring of general formula I, and R⁷ denotes a hydrogen atom,
the tautomers, the enantiomers, the diastereomers, the mixtures thereof and the salts thereof.

7. The following compounds of general formula I according to claim 1:
(a) 1-[(4-methyl-3-oxy-quinazolin-2-yl)methyl]-3-methyl-7-(2-butyn-1-yl)-8-((R)-3-amino-piperidin-1-yl)-xanthine,
(b) 1-[(1-oxy-quinolin-2-yl)methyl]-3-methyl-7-(2-butyn-1-yl)-8-((R)-3-amino-piperidin-1-yl)-xanthine,
(c) 1-[(3-methyl-2-oxy-isoquinolin-1-yl)methyl]-3-methyl-7-(2-butyn-1-yl)-8-((*R*)-3-amino-piperidin-1-yl)-xanthine,
(d) 1-[(5-oxy-phenanthridin-6-yl)methyl]-3-methyl-7-(2-butyn-1-yl)-8-((R)-3-amino-piperidin-1-yl)-xanthine,
(e) 1-[(3-oxy-quinazolin-2-yl)methyl]-3-methyl-7-(2-butyn-1-yl)-8-((*R*)-3-amino-piperidin-1-yl)-xanthine,
(f) 2-((*R*)-3-amino-piperidin-1-yl)-3-(2-butyn-1-yl)-5-[(1-oxy-quinolin-2-yl)methyl)]-3,5-dihydro-imidazo[4,5-d]pyridazin-4-one,
as well as the tautomers, the enantiomers, the diastereomers, the mixtures thereof and the salts thereof.

8. Physiologically acceptable salts of the compounds according to at least one of claims 1 to 7 with inorganic or organic acids.

9. Medicaments containing a compound according to at least one of claims 1 to 7 or a physiologically acceptable salt according to claim 8, optionally together with one or more inert carriers and/or diluents.

10. Use of a compound according to at least one of claims 1 to 8 for preparing a medicament which is suitable for treating type I and II diabetes mellitus, arthritis, obesity, allograft transplantation and calcitonin-induced osteoporosis.

11. Process for preparing a medicament according to claim 9, **characterised in that** a compound according to at least one of claims 1 to 8 is incorporated in one or more inert carriers and/or diluents by a non-chemical method.

12. Process for preparing the compounds of general formula I according to claims 1 to 8, **characterised in that**
a) a compound of general formula 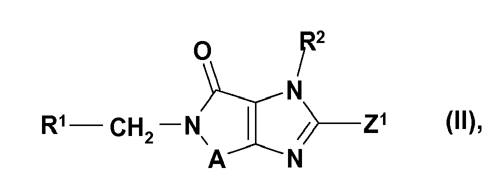 wherein
R¹, R² and A are defined as mentioned in claims 1 to 7 and
Z¹ denotes a leaving group such as a halogen atom, a substituted hydroxy, mercapto, sulphinyl, sulphonyl or sulphonyloxy group,
is reacted with R³-H, the enantiomers or the salts thereof, where R³ is as hereinbefore defined, or
b) a compound of general formula 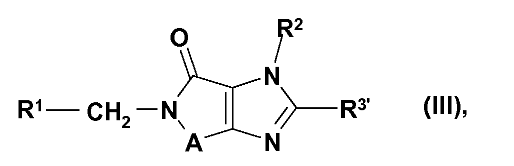 wherein R¹, R² and A are defined as mentioned in claims 1 to 7, and R^{3'} denotes one of the groups mentioned in the definition of R³ hereinbefore wherein the amino or imino group is protected by a protecting group, is deprotected,
and/or
any protecting groups used during the reaction are then cleaved and/or
the compounds of general formula I thus obtained are resolved into their enantiomers and/or diastereomers and/or
the compounds of formula I thus obtained are converted into their salts, particularly for pharmaceutical use into the physiologically acceptable salts thereof with inorganic or organic acids.

## Revendications

1. Composés de formule générale 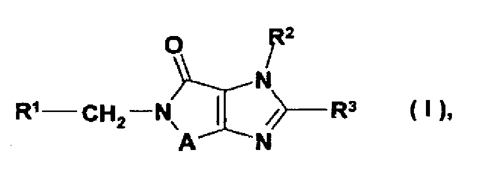 dans lesquels
R¹ représente un groupe pyridinyle, phénylpyridinyle, (pyridinylphényl)carbonyle, quinolinyle, phénylquinolinyle, isoquinolinyle, phénylisoquinolinyle ou phénanthridinyle substitué par les groupements R¹⁰ et R¹¹, où l'atome d'azote des groupes cités précédemment est substitué par un atome d'oxygène, et
R¹⁰ et R¹¹, qui peuvent être identiques ou différents, représentent un atome d'hydrogène, un atome de fluor, de chlore ou de brome ou un groupe méthyle, difluorométhyle, trifluorométhyle, méthoxy, difluorométhoxy, trifluorométhoxy ou cyano,
ou un groupe pyrimidinyle, phénylpyrimidinyle, (pyrimidinylphényl)carbonyle, quinazolinyle, phénylquinazolinyle, quinoxalinyle, phénylquinoxalinyle ou naphtyridinyle, où au moins un atome d'azote des groupes cités précédemment est substitué par un atome d'oxygène, et R¹⁰ et R¹¹ sont définis comme indiqué précédemment,
R² représente un groupe 2-butyn-1-yle,
R³ représente un groupe 3-aminopipéridin-1-yle, pipérazin-1-yle ou [1,4]-diazépan-1-yle, ou un groupe amino substitué par les groupements R⁴ et R⁵, où
R⁴ représente un groupe méthyle ou éthyle et
R⁵ représente un groupe 2-aminoéthyle, où la partie éthyle du groupe 2-aminoéthyle peut être substituée par un ou deux groupes méthyle,
et A représente un groupe -CO-N(R⁶)- où l'atome d'azote de ce groupe est lié au cycle imidazo de la formule générale I, et
R⁶ représente un groupe méthyle, éthyle, propyle, isopropyle, cyclopropyle ou phényle,
ou un groupe -N=C(R⁷)- où l'atome de carbone de ce groupe est lié au cycle imidazo de la formule générale I, et
R⁷ représente un atome d'hydrogène ou un groupe méthyle, leurs tautomères, énantiomères, diastéréoisomères, leurs mélanges et leurs sels.

2. Composés de formule générale I selon la revendication 1 dans lesquels
R¹ représente un groupe phénylpyridinyle, quinolinyle, isoquinolinyle ou phénanthridinyle substitué par les groupements R¹⁰ et R¹¹, où l'atome d'azote des groupes cités précédemment est substitué par un atome d'oxygène, et
R¹⁰ représente un atome d'hydrogène ou un groupe méthyle, méthoxy ou cyano et
et R¹¹ représente un atome d'hydrogène ou un groupe méthyle,
ou un groupe phénylpyrimidinyle, quinazolinyle, quinoxalinyle ou naphtyridinyle substitué par les groupements R¹⁰ et R¹¹, où au moins un atome d'azote des groupes cités précédemment est substitué par un atome d'oxygène, et R¹⁰ et R¹¹ sont définis comme indiqué précédemment,
R² représente un groupe 2-butyn-1-yle,
R³ représente un groupe 3-aminopipéridin-1-yle, pipérazin-1-yle ou [1,4]-diazépan-1-yle, ou un groupe amino substitué par les groupements R⁴ et R⁵, où
R⁴ représente un groupe méthyle et
R⁵ représente un groupe 2-aminoéthyle, où la partie éthyle du groupe 2-aminoéthyle peut être substituée par un ou deux groupes méthyle,
et A représente un groupe -CO-N(R⁶)- où l'atome d'azote de ce groupe est lié au cycle imidazo de la formule générale I, et
R⁶ représente un groupe méthyle, éthyle, isopropyle, cyclopropyle ou phényle,
ou un groupe -N=C(R⁷)- où l'atome de carbone de ce groupe est lié au cycle imidazo de la formule générale I, et
R⁷ représente un atome d'hydrogène ou un groupe méthyle,
leurs tautomères, énantiomères, diastéréoisomères, leurs mélanges et leurs sels.

3. Composés selon au moins l'une des revendications 1 à 2 dans lesquels R¹, R² et A sont définis comme indiqué dans les revendications 1 à 2 et R³ représente un groupe 3-aminopipéridin-1-yle, leurs tautomères, énantiomères, diastéréoisomères, leurs mélanges et leurs sels.

4. Composés selon au moins l'une des revendications 1 à 2 dans lesquels R¹, R² et A sont définis comme indiqué dans les revendications 1 à 2 et R³ représente un groupe pipérazin-1-yle, leurs tautomères, énantiomères, diastéréoisomères, leurs mélanges et leurs sels.

5. Composés selon au moins l'une des revendications 1 à 2 dans lesquels R¹, R² et A sont définis comme indiqué dans les revendications 1 à 2 et R³ représente un groupe [1,4]-diazépan-1-yle, leurs tautomères, énantiomères, diastéréoisomères, leurs mélanges et leurs sels.

6. Composés de formule générale I selon la revendication 1 dans lesquels
R¹ représente un groupe quinolinyle, isoquinolinyle, méthylisoquinolinyle ou phénanthridinyle, où l'atome d'azote des groupes cités précédemment est substitué par un atome d'oxygène,
un groupe quinazolinyle ou méthylquinazolinyle, où un atome d'azote des groupes cités précédemment est substitué par un atome d'oxygène,
ou un groupe quinoxalinyle dans lequel les deux atomes d'azote sont substitués par des atomes d'oxygène,
R² représente un groupe 2-butyn-1-yle,
R³ représente un groupe 3-aminopipéridin-1-yle ou un groupe pipérazin-1-yle et A représente un groupe -CO-N(R⁶)- où l'atome d'azote de ce groupe est lié au cycle imidazo de la formule générale I, et R⁶ représente un groupe méthyle,
ou un groupe -N=C(R⁷)- où l'atome de carbone de ce groupe est lié au cycle imidazo de la formule générale I, et R⁷ représente un atome d'hydrogène,
leurs tautomères, énantiomères, diastéréoisomères, leurs mélanges et leurs sels.

7. Composés de formule générale I selon la revendication 1 suivants :
(a) 1-[(4-méthyl-3-oxy-quinazolin-2-yl)méthyl]-3-méthyl-7-(2-butyn-1-yl)-8-((R)-3-amino-pipéridin-1-yl)-xanthine,
(b) 1-[(1-oxy-quinolin-2-yl)méthyl]-3-méthyl-7-(2-butyn-1-yl)-8-((R)-3-amino-pipéridin-1-yl)-xanthine,
(c) 1-[(3-méthyl-2-oxy-isoquinolin-1-yl)méthyl]-3-méthyl-7-(2-butyn-1-yl)-8-((*R*)-3-amino-pipéridin-1-yl)-xanthine,
(d) 1-[(5-oxy-phénanthridin-6-yl)méthyl]-3-méthyl-7-(2-butyn-1-yl)-8-((R)-3-amino-pipéridin-1-yl)-xanthine,
(e) 1-[(3-oxy-quinazolin-2-yl)méthyl]-3-méthyl-7-(2-butyn-1-yl)-8-((*R*)-3-amino-pipéridin-1-yl)-xanthine,
(f) 2-((*R*)-3-amino-pipéridin-1-yl)-3-(2-butyn-1-yl)-5-[(1-oxy-quinolin-2-yl)méthyl)]-3,5-dihydro-imidazo[4,5-d]pyridazin-4-one,
ainsi que leurs tautomères, énantiomères, diastéréoisomères, leurs mélanges et leurs sels.

8. Sels physiologiquement acceptables des composés selon au moins l'une des revendications 1 à 7 avec des acides inorganiques ou organiques.

9. Médicament contenant un composé selon au moins l'une des revendications 1 à 7 ou un sel physiologiquement acceptable selon la revendication 8 outre éventuellement un ou plusieurs vecteurs et/ou diluants inertes.

10. Utilisation d'un composé selon au moins l'une des revendications 1 à 8 pour la production d'un médicament qui convient pour le traitement du diabète sucré de type I et de type II, de l'arthrite, de l'adiposité, de la transplantation d'allogreffes et de l'ostéoporose provoquée par la calcitonine.

11. Procédé pour la production d'un médicament selon la revendication 9 **caractérisé en ce que,** par voie non chimique, un composé selon au moins l'une des revendications 1 à 8 est incorporé dans un ou plusieurs vecteurs et/ou diluants inertes.

12. Procédé pour la production des composés de formule générale I selon les revendications 1 à 8 **caractérisé en ce que**
a) un composé de formule générale 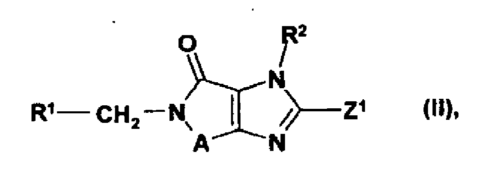 dans lequel
R¹, R² et A sont définis comme indiqué dans les revendications 1 à 7 et
Z¹ représente un groupe partant comme un atome d'halogène, un groupe hydroxy, mercapto, sulfinyle, sulfonyle ou sulfonyloxy substitué,
est mis à réagir avec R³-H, ses énantiomères ou ses sels, où R³ est défini comme indiqué au début, ou
b) un composé de formule générale 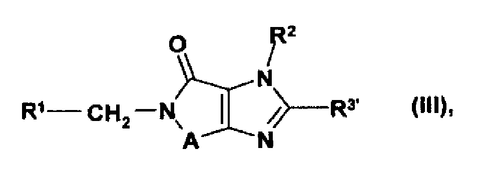 dans lequel R¹, R² et A sont définis comme indiqué dans les revendications 1 à 7 et R^{3'} représente l'un des groupes définis au début pour R³, dans lesquels le groupe amino ou imino est protégé par un groupe protecteur, est déprotégé,
et/ou
les groupes protecteurs éventuellement utilisés pendant la réaction sont ensuite clivés et/ou
les composés de formule générale I ainsi obtenus sont séparés en leurs énantiomères et/ou diastéréoisomères et/ou
les composés de formule I obtenus sont convertis en leurs sels, en particulier pour l'utilisation pharmaceutique en leurs sels physiologiquement acceptables avec des acides inorganiques ou organiques.
